# EUROPEAN PATENT SPECIFICATION

(11) **EP 0 639 559 B1**
(45) Date of publication and mention of the grant of the patent: **15.10.1997**
(21) Application number: 94305803.2
(22) Date of filing: 04.08.1994
(51) Int. Cl.: C07C 243/38

(54) **Preparation of 1,2-diacyl-2-(t-alkyl)hydrazines**
Die Herstellung von 1,2-Diacyl-2-(t-alkyl)-Hydrazinen
La préparation des 1,2-diacyl-2-(t-alkyle)hydrazines

(30) Priority: 19.08.1993 US 109116
(43) Date of publication of application: 22.02.1995
(73) Proprietor: ROHM AND HAAS COMPANY, Philadelphia, Pennsylvania 19106-2399 (US)
(72) Inventor: Kelly, Martha Jean, Norristown, Pennsylvania 19403 (US)
(74) Representative: Tanner, James Percival

(56) References cited:
- EP-A- 0 236 618
- EP-A- 0 483 647
- EP-A- 0 492 839
- Derivatives of hydrazine and other hydronitrogens having N-N bonds,The Benjamin/Cummings Publishing Compagny, Reading,MA (1983),pp 11-12
- Ullmann's Encyclopedia of Indusrial Chemistry,5th Edition,(1989),Vol. A13,p.188

## Description

The present invention is concerned with a process for preparing 1,2-diacyl-2-(*t*-alkyl)hydrazines. More particularly, the present invention is concerned with a process for preparing the aforesaid diacylhydrazines, utilizing a solvent comprising an ester or a mixture of an ester and water, and in which process an aromatic acid chloride is reacted in a first step with a *t*-alkylhydrazine or a corresponding acid addition salt of a t-alkylhydrazine such as the hydrochloride salt in the presence of a base to afford a 1-acyl-2-*t*-alkylhydrazine followed by a second step wherein another aromatic acid chloride is reacted with the aforesaid monoacylhydrazine in the presence of a base to afford the desired 1,2-diacyl-2-(*t*-alkyl)hydrazine. Such compounds are known to have excellent insecticidal activity against insects of the orders Lepidoptera and Coleoptera.

The search for process solvents which are environmentally friendly, which are easily removed from the desired product, which provide good selectivity to the desired intermediates and products, which are easily recovered from the desired products, which allow a facile purification of the desired products, which possess favorable solubility properties for both the reactants and the desired intermediates and products, which can be used in all steps of the process, which are inert to the reaction conditions and which possess favorable process economics is a continuing one because of the difficulty in finding solvents or mixtures of solvents which fulfill all these important conditions. For example, US-A-4,985,461 discloses a process leading to 1,2-diacyl-2-(*t*-alkyl)hydrazines which employs one or more solvents such as water, alcohols such as methanol, ethanol and isopropanol, hydrocarbons such as toluene, xylene, hexane and heptane, glyme, tetrahydrofuran, acetonitrile, pyridine, haloalkanes such as methylene chloride or mixtures of these solvents. However, all these solvents suffer from one or more of the deficiencies previously noted. For example, water by itself results in poor intermediate and product yields and poor selectivity with a large amount of diacylation of the *t*-butylhydrazine occurring in the first process step. Alcohols such as methanol, ethanol and isopropanol react with the acid chlorides, thus resulting in undesirable consumption of the acid chlorides and complicating the recovery and recycle of the process solvents. Hydrocarbons such as toluene, xylene, hexane and heptane provide poor reaction selectivity in the first process step with resultant unacceptable levels of the wrong acylation isomer; additionally, these solvents are carefully regulated by governmental authorities because of environmental and safety concerns. Glyme and tetrahydrofuran are both ethers which can form undesirable levels of dangerous peroxides upon continued recovery and recycle to the process; they are also quite water soluble which further increases the difficulty of their recovery. Acetonitrile and pyridine not only are both very water soluble and hence difficult to recover in an aqueous system, but also both possess toxicological and safety concerns. Haloalkanes such as methylene chloride have a high vapor pressure which results in difficulty to contain and prevent emissions to the environment; the solubility of usual monoacylhydrazine intermediates and diacylhydrazine products is also usually very high in such solvents which results in problems to recover the aforesaid intermediates and products efficiently from such solvents.

It is, therefore, an aim of the present invention to provide a solvent which can be used in both steps of the process leading to 1,2-diacyl-2-(*t*-alkyl)hydrazines, which is environmentally friendly, which provides good selectivity, purities and yields for the aforesaid intermediates and products, which is easily recovered and recycled in the process, which allows facile removal of the product after completion of the reactions leading to it, which does not participate in the reaction process to provide undesirable side products, and which is economical to utilize. It has now unexpectedly been found that esters, more particularly aliphatic esters, possess these desirable attributes. Although US-A-4,985,461 teaches the use of the solvents listed previously, it does not teach or suggest the use of esters, with their attendant advantages, as solvents in a process to make 1,2-diacyl-2-(*t*-alkyl)hydrazines.

According to the present invention, there is provided a process, for the preparation of pesticidal 1,2-diacyl-2-(*t*-alkyl)hydrazine compounds of the formula 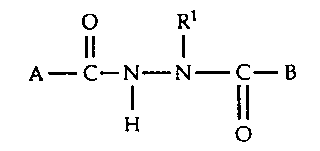 wherein
R¹ is a tertiary (C₄-C₈)alkyl group,
A and B are each independently (i) phenyl, (ii) naphthyl, or (iii) phenyl or naphthyl substituted with one to three of the same or different substituents selected from the group consisting of: halo; cyano; nitro; hydroxy; mercapto; thiocyanato; (C₁-C₄)alkyl; (C₁-C₄)alkoxy; halo(C₁-C₂)alkyl; halo(C₁-C₂)alkoxy; (C₁-C₄)alkylthio; (C₁-C₄)alkylsulfinyl; (C₁-C₄)alkylsulfonyl; carboxy; formyl; (C₁-C₄)alkylcarbonyl; (C₁-C₄)alkoxycarbonyl; (C₁-C₄)alkanoyloxy; amino; (C₁-C₄)alkylamino; di(C₁-C₄)alkylamino having independently the stated number of carbon atoms in each alkyl group; carbamoyl; (C₁-C₄)alkylcarbamoyl; di(C₁-C₄)alkylcarbamoyl having independently the stated number of carbon atoms in each alkyl group; cyano(C₁-C₄)alkyl; (C₁-C₄)alkoxy(C₁-C₄)alkyl; (C₂-C₆)alkenyl; (C₄-C₆)alkadienyl; (C₂-C₆)alkynyl; (C₁-C₄)alkyldithionate; (C₁-C₄)alkylcarbonylthio; tri(C₁-C₄)alkylsilyl having independently the stated number of carbon atoms in each alkyl group; phenyl; phenoxy; benzoyl; phenoxycarbonyl; phenylthio; phenyl(C₁-C₄)alkyl; and wherein the phenyl, phenoxy, benzoyl, phenoxycarbonyl, phenylthio and phenyl(C₁-C₄)alkyl substituents are optionally substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; or when two adjacent positions on a phenyl ring are substituted with alkoxy groups, these groups may be joined to form a 5 or 6 membered dioxolano (methylenedioxy) or dioxano (1,2-ethylenedioxy) heterocyclic ring;
or the agronomically acceptable salts thereof;
whereby said process comprises:
(a) reacting an aromatic acid chloride of the formula: 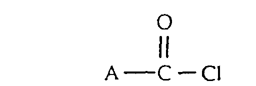 with a tertiary (C₄-C₈)alkylhydrazine of the formula:

   H₂NNHR¹

   or a corresponding acid addition salt of said tertiary (C₄-C₈)alkylhydrazine in the presence of a base, at a temperature of from -20°C to 40°C, to form an intermediate 1-acyl-2-(*t*-alkyl)hydrazine of the formula: 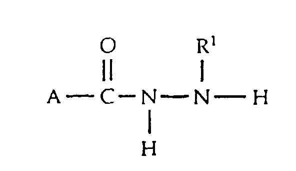 wherein A and R¹ have the same definitions as previously; and
(b) reacting said intermediate hydrazine from step (a) with an aromatic acid chloride of the formula : 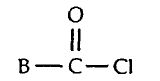 in the presence of a base, at a temperature of from 25°C to 100°C, to form the said 1,2-diacyl-2-(*t*-alkyl)hydrazine, wherein B has the same definitions as previously; and
characterized in that the process is carried out utilising a solvent comprising an ester or a mixture of an ester and water.

As used herein:-
Halo is chloro, fluoro, bromo or iodo;
(C₁-C₄)alkyl is a straight chain or branched chain alkyl group;
Tertiary (C₄-C₈)alkyl is, for example, *t*-butyl, 1,1-dimethylpentyl or 1,1,3,3-tetramethylbutyl;
(C₂-C₆)alkenyl is, for example, vinyl, allyl or 2-buten-1-yl;
(C₄-C₆)alkadienyl is, for example, 2,4-pentadien-1-yl;
(C₂-C₆)alkynyl is, for example, propargyl;
(C₁-C₄)alkoxy is, for example, methoxy, ethoxy or isopropoxy;
(C₁-C₄)alkoxy(C₁-C₄)alkyl is, for example, methoxymethyl or 2-methoxyethyl;
Halo(C₁-C₂)alkyl is, for example, trifluoromethyl, 1,1,2,2,2-pentafluoroethyl or chloromethyl;
Halo(C₁-C₂)alkoxy is, for example, difluoromethoxy;
(C₁-C₄)alkylthio is, for example, methylthio;
(C₁-C₄)alkylcarbonyl is, for example, methylcarbonyl (acetyl);
(C₁-C₄)alkoxycarbonyl is, for example, ethoxycarbonyl;
(C₁-C₄)alkanoyloxy is, for example, methylcarbonyloxy (acetoxy);
(C₁-C₄)alkylamino is, for example, isobutylamino.;
Di(C₁-C₄)alkylamino is, for example, dimethylamino or N-methyl-N-ethylamino;
(C₁-C₄)alkylcarbamoyl is, for example, *n*-butylcarbamoyl;
Di(C₁-C₄)alkylcarbamoyl is, for example, dimethylcarbamoyl or N-methyl-N-ethylcarbamoyl;
(C₁-C₄)alkylsulfinyl is, for example, methylsulfinyl.;
(C₁-C₄)alkylsulfonyl is, for example, methylsulfonyl;
(C₁-C₄)alkyldithionate is, for example, methyldithionate;
(C₁-C₄)alkylcarbonylthio is, for example, ethylcarbonylthio;
Tri(C₁-C₄)alkylsilyl is, for example, trimethylsilyl or diethylmethylsilyl;
Phenyl(C₁-C₄)alkyl is, for example, benzyl or 4-chlorophenethyl;
Methylenedioxy is the -OCH₂O- moiety; and
1,2-Ethylenedioxy is the -OCH₂CH₂O- moiety.

Typical acid chlorides which may be used in the process of the present invention include, but are not limited to, benzoyl chloride, 3-methylbenzoyl chloride, 3,5-dimethylbenzoyl chloride, 3,5-dichlorobenzoyl chloride, 4-ethylbenzoyl chloride, 4-chlorobenzoyl chloride, 2-methyl-3-methoxybenzoyl chloride, 3,5-dichlorobenzoyl chloride, 3-chlorobenzoyl chloride, 4-cyanobenzoyl chloride, 3-nitrobenzoyl chloride, 2,3-dimethylbenzoyl chloride, 3-methoxybenzoyl chloride, 3-fluorobenzoyl chloride, 4-(t-butyl)benzoyl chloride, 2,4-dichlorobenzoyl chloride, 3-(trifluoromethyl)benzoyl chloride, 2-acetoxybenzoyl chloride, 4-(4-chlorophenethyl)benzoyl chloride, 1-naphthoyl chloride, 4-isopropylbenzoyl chloride, 2,6-difluorobenzoyl chloride, 2-(difluoromethoxy)benzoyl chloride, 4-acetylbenzoyl chloride, 3-(dimethylamino)benzoyl chloride, 2-nitro-4-((4-trifluoromethyl)-2-chlorophenoxy)benzoyl chloride, phenylthiobenzoyl chloride, 4-(2-fluorobenzyl)benzoyl chloride, 4-(4-chlorobenzoyl)benzoyl chloride, 4-(phenoxycarbonyl)benzoyl chloride, 2-methyl-3,4-(1,2-ethylenedioxy)benzoyl chloride, and 2-methyl-3,4-(methylenedioxy)benzoyl chloride.

Typical tertiary alkylhydrazines which may be used in the process of the present invention include, but are not limited to, *t*-butylhydrazine, 1,1-dimethylpentylhydrazine and 1,1,3,3-tetramethylbutylhydrazine, and their corresponding acid addition salts such as the hydrochloride salt, for example, *t*-butylhydrazine hydrochloride.

Typical 1,2-diacyl-2-(t-alkyl)hydrazine products which may be produced by the process of this invention include, but are not limited to, N'-*t*-butyl-N,N'-dibenzoylhydrazine, N'-*t*-butyl-N-(4-chlorobenzoyl)-N'-benzoylhydrazine, N'-*t*-butyl-N-(4-ethylbenzoyl)-N'-(3,5-dimethylbenzoyl)hydrazine, N'-*t*-butyl-N-(2-methyl-3-methoxybenzoyl)-N'-(3,5-dimethylbenzoyl)hydrazine, N'-*t*-butyl-N-(4-ethylbenzoyl)-N'-(3-methylbenzoyl)hydrazine, N'-*t*-butyl-N-(2-ethyl-3-methoxybenzoyl)-N'-(3,5-dimethylbenzoyl)hydrazine, N'-*t*-butyl-N-(2,3-dimethylbenzoyl)-N'-(3,5-dimethylbenzoyl)hydrazine, N'-*t*-butyl-N-(2,3-dimethylbenzoyl)-N'-(3-methylbenzoyl)hydrazine, N'-*t*-butyl-N-(4-ethylbenzoyl)-N'-(3,5-dichlorobenzoyl)hydrazine, N'-*t*-butyl-N-(2-methyl-3,4-(1,2-ethylenedioxy)benzoyl)-N'-(3,5-dimethylbenzoyl)hydrazine, N'-*t*-butyl-N-(2-methyl-3,4-(methylenedioxy)benzoyl)-N'-(3,5-dimethylbenzoyl)hydrazine, N'-*t*-butyl-N-(2-fluorobenzoyl)-N'-benzoylhydrazine, N'-*t*-butyl-N-(1-naphthoyl)-N'-(3,5-dimethylbenzoyl)hydrazine, N'-(1,1-dimethylpentyl)-N-(4-chlorobenzoyl)-N'-benzoylhydrazine and N'-(1,1,3,3-tetramethylbutyl)-N-(2-methyl-3-methoxybenzoyl)-N'-(3,5-dimethylbenzoyl)hydrazine.

Preferably, 1,2-diacyl-2-(t-alkyl) hydrazine compounds, produced by the process of the present invention, are selected from the group consisting of:
N'-*t*-butyl-N,N'-dibenzoylhydrazine,
N'-*t*-butyl-N-(2-methyl-3-methoxybenzoyl)-N'-(3,5-dimethylbenzoyl)hydrazine,
N'-*t*-butyl-N-(4-ethylbenzoyl)-N'-(3,5-dimethylbenzoyl)hydrazine and
N'-*t*-butyl-N-(4-chlorobenzoyl)-N'-benzoylhydrazine

Typical esters which may be utilized as solvents or cosolvents with water in the process of this invention include, but are not limited to, (C₁-C₄)alkyl (C₁-C₅)alkanoates such as *n*-butyl acetate, methyl *n*-butyrate, ethyl isopropionate, *n*-propyl acetate, isopropyl acetate, ethyl acetate, methyl pentanoate, isobutyl acetate and ethyl *n*-butyrate.

In the process of the present invention, when A and B are the same, for example, both A and B are 4-chlorophenyl, two equivalents of the aromatic acid chloride of the formula: 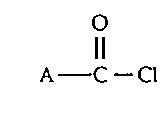 may be reacted with a tertiary (C₄-C₈)alkylhydrazine of the formula:

H₂NNHR¹

or a corresponding acid addition salt of the tertiary (C₄-C₈)alkylhydrazine in the presence of a base in a solvent comprising an ester or a mixture of water and an ester solvent to form the desired 1,2-diacyl-2-(*t*-alkyl)hydrazine product having the formula: 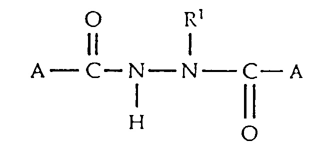 wherein A and R¹ are the same as defined previously.

Examples of bases for use in the process of the present invention include, but are not limited to, tertiary amines such as triethylamine, pyridine, potassium carbonate, sodium carbonate, sodium bicarbonate, potassium bicarbonate, sodium hydroxide, potassium hydroxide, or mixtures thereof.

The process of the present invention can be carried out at temperatures of from -20° C to 100° C at about atmospheric pressure, although higher or lower pressures can be used if desired. More specifically, the temperatures utilized in the first step (step a) of the present process are in the range of from -20° C to 40° C, and the temperatures utilized in the second step (step b) of the present process are in the range of from 25° C to 100° C.

Typically, substantially equimolar amounts of reactants are used in the process although higher or lower amounts can be used if desired. Generally, from about 1.0 to about 1.5 equivalents of base are used per equivalent of the aromatic acid chloride starting material. When an acid addition salt of the tertiary alkylhydrazine is employed, one additional equivalent of base is typically employed.

Modifications to the process of the present invention may be necessary to accommodate reactive functionalities of particular A and/or B substituents. Such modifications would be apparent and known to those skilled in the art. Procedures for making the agronomically acceptable salts embraced by the products resulting from the process of the present invention are also well known to those skilled in the art and have been previously described in the literature, e.g. in US-A-4,985,461.

In a preferred embodiment of the present invention, there is provided a process, utilizing a solvent comprising a (C₂-C₄)alkyl (C₂-C₅)alkanoate ester or a mixture of a (C₂-C₄)alkyl (C₂-C₅)alkanoate ester and water, for the preparation of 1,2-diacyl-2-(*t*-alkyl)hydrazine compounds of the formula: 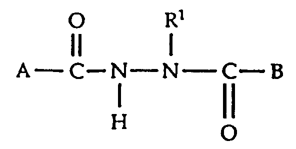 wherein:
R¹ is *t*-butyl, 1,1-dimethylpentyl or 1,1,3,3-tetramethylbutyl;
A and B are each independently (i) phenyl, (ii) naphthyl, or (iii) phenyl or naphthyl substituted with one to three of the same or different substituents selected from the group consisting of:halo; cyano; nitro; thiocyanato; (C₁-C₄)alkyl; (C₁-C₄)alkoxy; halo(C₁-C₂)alkyl; halo(C₁-C₂)alkoxy; (C₁-C₄)alkylthio; (C₁-C₄)alkylsulfinyl; (C₁-C₄)alkylsulfonyl; carboxy; formyl; (C₁-C₄)alkylcarbonyl; (C₁-C₄)alkoxycarbonyl; (C₁-C₄)alkanoyloxy; di(C₁-C₄)alkylamino having independently the stated number of carbon atoms in each alkyl group; carbamoyl; (C₁-C₄)alkylcarbamoyl; di(C₁-C₄)alkylcarbamoyl having independently the stated number of carbon atoms in each alkyl group; cyano(C₁-C₄)alkyl; (C₁-C₄)alkoxy(C₁-C₄)alkyl; (C₂-C₆)alkenyl; (C₄-C₆)alkadienyl; (C₂-C₆)alkynyl; (C₁-C₄)alkyldithionate; (C₁-C₄)alkylcarbonylthio; tri(C₁-C₄)alkylsilyl having independently the stated number of carbon atoms in each alkyl group; phenyl; phenoxy; benzoyl; phenyl(C₁-C₂)alkyl or phenyl(C₁-C₂)alkyl substituted on the phenyl ring with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, di(C₁-C₄)alkylamino having independently the stated number of carbon atoms in each alkyl group; or when two adjacent positions on a phenyl ring are substituted with alkoxy groups, these groups may be joined to form a 5 or 6 membered dioxolano or dioxano heterocyclic ring;
or the agronomically acceptable salts thereof;
whereby said process comprises:
(a) reacting an aromatic acid chloride of the formula: 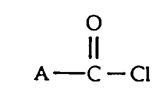 with *t*-butylhydrazine, 1,1-dimethylpentylhydrazine or 1,1,3,3-tetramethylbutylhydrazine or a corresponding acid addition salt of said hydrazine in the presence of potassium hydroxide, sodium hydroxide, sodium carbonate, potassium carbonate, sodium bicarbonate, potassium bicarbonate or a mixture thereof at a temperature from -5° C to 40° C to form an intermediate 1-acyl-2-(*t*-alkyl)hydrazine of the formula: 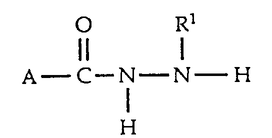 wherein A and R¹ have the same definitions as previously; and
(b) reacting said intermediate hydrazine from step (a) with an aromatic acid chloride of the formula: 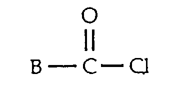 in the presence of potassium hydroxide, sodium hydroxide, sodium carbonate, potassium carbonate, sodium bicarbonate, potassium bicarbonate or a mixture thereof at a temperature of from 25° C to 95° C to form the said 1,2-diacyl-2-(*t*-alkyl)hydrazine, wherein B has the same definitions as previously.

In a more preferred embodiment of the present invention, there is provided a process, utilizing a solvent comprising a mixture of water and an ester selected from the group consisting of isobutyl acetate, ethyl acetate, isopropyl acetate and *n*-butyl acetate, for the preparation of 1,2-diacyl-2-(*t*-butyl)hydrazine compounds of the formula: 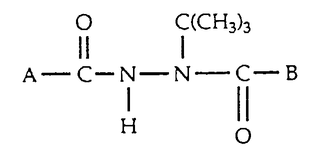 wherein:
A and B are each independently phenyl, or phenyl substituted with one to three of the same or different substituents selected from the group consisting of halo, (C₁-C₂)alkyl, (C₁-C₂)alkoxy, (C₁-C₂)alkoxy(C₁-C₂)alkyl ,or when two adjacent positions on a phenyl ring are substituted with alkoxy groups, these groups may be joined to form a 5 or 6 membered dioxolano or dioxano heterocyclic ring;
whereby said process comprises:
(a) reacting an aromatic acid chloride of the formula: 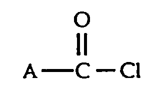 with t-butylhydrazine or *t*-butylhydrazine hydrochloride in the presence of potassium hydroxide, sodium hydroxide, sodium carbonate, sodium bicarbonate, potassium carbonate, or a mixture thereof to form an intermediate 1-acyl-2-(*t*-butyl)hydrazine of the formula: 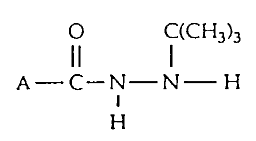 wherein A has the same definitions as previously; and
(b) reacting said intermediate hydrazine from step (a) with an aromatic acid chloride of the formula: 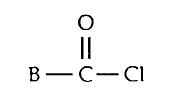 in the presence of sodium carbonate, sodium bicarbonate or potassium carbonate or a mixture thereof to form the said 1,2-diacyl-2-(*t*-butyl)hydrazine, wherein B has the same definitions as previously.

In a most preferred embodiment of the present invention, there is provided a process, utilizing a solvent comprising a mixture of water and *n*-butyl acetate as solvent, for the preparation of 1,2-diacyl-2-(*t*-butyl)hydrazine compounds of the formula: 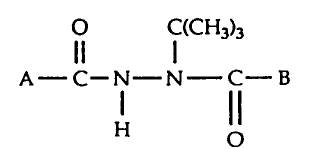 wherein:
A is phenyl, 2-fluorophenyl, 4-chlorophenyl, 2-methyl-3-methoxyphenyl or 4-ethylphenyl;
B is phenyl, 3,5-dimethylphenyl, 3-methylphenyl, 3-chlorophenyl or 3,5-dichlorophenyl;
whereby said process comprises:
(a) reacting an aromatic acid chloride of the formula: 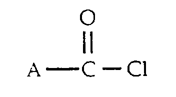 with *t*-butylhydrazine hydrochloride in the presence of a mixture of sodium hydroxide and potassium carbonate to form an intermediate 1-acyl-2-(*t*-butyl)hydrazine of the formula: 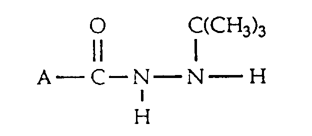 wherein A has the same definitions as previously; and
(b) reacting said intermediate hydrazine from step (a) with an aromatic acid chloride of the formula: 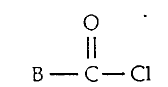 in the presence of sodium carbonate or potassium carbonate to form the said 1,2-diacyl-2-(*t*-butyl)hydrazine, wherein B has the same definitions as previously.

The following Examples are presented to illustrate various embodiments of the process of the present invention.

### EXAMPLE 1: PREPARATION OF N'-t-BUTYL-N-(4-ETHYLBENZOYL)-N'-(3,5-DIMETHYLBENZOYL)HYDRAZINE

**STEP A:** To a flask inerted with nitrogen was added 65.4 grams (g) of *t*-butylhydrazine hydrochloride and 50 g of water. The temperature was maintained at 20° C or lower as 78.8 g of 20% sodium hydroxide solution was charged while stirring the flask contents. This was followed by the addition of a solution of 36.2 g of potassium carbonate in 50 g of water. The reaction mixture was cooled to 15° C and 300 g of *n*-butyl acetate was added. While continuing stirring at 15° C, 84.3 g of 4-ethylbenzoyl chloride and 100g of 20% sodium hydroxide solution were cofed to the reaction mixture over a one hour period. The resulting slurry was held an additional 30 minutes at 15° C and was then heated to 40° C. The solids dissolved and the aqueous phase was removed. The *n*-butyl acetate phase was washed with 100 g of water and the wash discarded.

**STEP B:** To the *n*-butyl acetate phase was added 100 g of water and the resulting mixture was heated to 60° C. An aqueous potassium carbonate solution, which was prepared from 37.3 g of anhydrous potassium carbonate, and 84.3 g of 3,5-dimethylbenzoyl chloride were cofed to the stirred reaction mixture. When the feeds were 50% complete, the reaction mixture was seeded with 0.18 g of N'-t-butyl-N-(4-ethylbenzoyl)-N'-(3,5-dimethylbenzoyl)hydrazine. After the appearance of solids, the feeds were resumed. After completion of the feeds, the reaction mixture was stirred an additional hour at 60° C and then the phases were allowed to settle. The aqueous phase was discarded and the slurry was washed twice with 100 g portions of water at 60° C and then cooled to 5° C. The solids were filtered, washed with cold *n*-butyl acetate and then dried under vacuum resulting in the recovery of N'-t-butyl-N-(4-ethylbenzoyl)-N'-(3,5-dimethylbenzoyl)hydrazine as white solid of 96.5% purity.

### EXAMPLES 2-5:

By following substantially the same procedure as Example 1 and utilizing the aromatic acid chlorides listed for the steps as shown, the following compounds may be prepared as further illustrations of the process of this invention:

| **EX. NO.** | **STEP A** | **STEP B** | **PRODUCT** |
|---|---|---|---|
| 2 | benzoyl | benzoyl | N'-*t*-butyl-N,N'-dibenzoylhydrazine |
| 3 | 2-fluorobenzoyl | benzoyl | N'-*t*-butyl-N-(2-fluorobenzoyl)-N'-benzoylhydrazine |
| 4 | 4-chlorobenzoyl | benzoyl | N'-*t*-butyl-N-(4-chlorobenzoyl)-N'-benzoylhydrazine |
| 5 | 2-methyl-3-methoxybenzoyl | 3,5-dimethylbenzoyl | N'-*t*-butyl-N-(2-methyl-3-methoxybenzoyl)-N'-(3,5-dimethylbenzoyl)hydrazine |

## Claims

1. A process for the preparation of pesticidal 1,2-diacyl-2-(t-alkyl)hydrazine compounds of the formula: 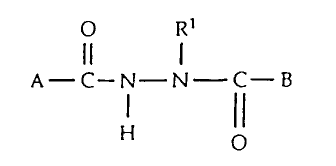 wherein:
R¹ is a tertiary (C₄-C₈)alkyl group;
A and B are each independently (i) phenyl, (ii) naphthyl, or (iii) phenyl or naphthyl substituted with one to three of the same or different substituents selected from the group consisting of: halo; cyano; nitro; hydroxy; mercapto; thiocyanato; (C₁-C₄)alkyl; (C₁-C₄)alkoxy; halo(C₁-C₂)alkyl; halo(C₁-C₂)alkoxy; (C₁-C₄)alkylthio; (C₁-C₄)alkylsulfinyl; (C₁-C₄)alkylsulfonyl; carboxy; formyl; (C₁-C₄)alkylcarbonyl; (C₁-C₄)alkoxycarbonyl; (C₁-C₄)alkanoyloxy; amino; (C₁-C₄)alkylamino; di(C₁-C₄)alkylamino having independently the stated number of carbon atoms in each alkyl group; carbamoyl; (C₁-C₄)alkylcarbamoyl; di(C₁-C₄)alkylcarbamoyl having independently the stated number of carbon atoms in each alkyl group; cyano(C₁-C₄)alkyl; (C₁-C₄)alkoxy(C₁-C₄)alkyl; (C₂-C₆)alkenyl; (C₄-C₆)alkadienyl; (C₂-C₆)alkynyl; (C₁-C₄)alkyldithionate; (C₁-C₄)alkylcarbonylthio; tri(C₁-C₄)alkylsilyl having independently the stated number of carbon atoms in each alkyl group; phenyl; phenoxy; benzoyl; phenoxycarbonyl; phenylthio; phenyl(C₁-C₄)alkyl; and wherein the phenyl, phenoxy, benzoyl, phenoxycarbonyl, phenylthio and phenyl(C₁-C₄)alkyl substituents are optionally substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; or when two adjacent positions on a phenyl ring are substituted with alkoxy groups, these groups may be joined to form a 5 or 6 membered dioxolano (methylenedioxy) or dioxano (1,2-ethylenedioxy) heterocyclic ring;
or the agronomically acceptable salts thereof;
whereby said process comprises:
(a) reacting an aromatic acid chloride of the formula: 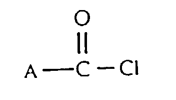 with a tertiary(C₄-C₈)alkylhydrazine of the formula:
H₂NNHR¹
or a corresponding acid addition salt of said tertiary (C₄-C₈)alkylhydrazine in the presence of a base, at a temperature of from -20°C to 40°C. to form an intermediate 1-acyl-2-(*t*-alkyl)hydrazine of the formula: 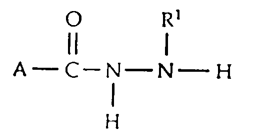 wherein A and R¹ have the same definitions as previously; and
(b) reacting said intermediate hydrazine from step (a) with an aromatic acid chloride of the formula 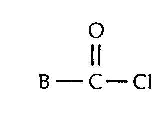 in the presence of a base, at a temperature of from 25°C to 100°C,, to form the said 1,2-diacyl-2-(t-alkyl)hydrazine, wherein B has the same definitions as previously; and characterised in that the process is carried out utilising a solvent comprising an ester or a mixture of an ester and water.

2. A process as claimed in claim 1, wherein the acid chloride, used in step (a) and/or step (b), is selected from: benzoyl chloride, 3-methylbenzoyl chloride, 3,5-dimethylbenzoyl chloride, 3,5-dichlorobenzoyl chloride, 4-ethylbenzoyl chloride, 4-chlorobenzoyl chloride, 2-methyl-3-methoxybenzoyl chloride, 3,5-dichlorobenzoyl chloride, 3-chlorobenzoyl chloride, 4-cyanobenzoyl chloride, 3-nitrobenzoyl chloride, 2,3-dimethylbenzoyl chloride, 2-methoxybenzoyl chloride, 3-fluorobenzoyl chloride, 4-(t-butyl)benzoyl chloride, 2,4-dichlorobenzoyl chloride, 3-(trifluoromethyl)benzoyl chloride, 2-acetoxybenzoyl chloride, 4-(4-chlorophenethyl)benzoyl chloride, 1-naphthoyl chloride, 4-isopropylbenzoyl chloride, 2,6-difluorobenzoyl chloride, 2-(difluoromethoxy)benzoyl chloride, 4-acetylbenzoyl chloride, 3-(dimethylamino)benzoyl chloride, 2-nitro-4-((4-trifluoromethyl)-2-chlorophenoxy)benzoyl chloride, phenylthiobenzoyl chloride, 4-(2-fluorobenzyl)benzoyl chloride, 4-(4-chlorobenzoyl) chloride, 4-(phenoxycarbonyl)benzoyl chloride, 2-methyl-3,4-(1,2-ethylenedioxy)benzoyl chloride and 2-methyl-3,4-(methylenedioxy)benzoyl chloride.

3. A process as claimed in claim 1 or claim 2, wherein the tertiary (C₄-C₈) alkylhydrazine, used in step (a) in selected from: t-butylhydrazine, 1,1-dimethylpentylhydrazine and 1,1,3,3-tetramethylbutylhydrazine, and their corresponding acid addition salts.

4. A process as claim in claim 1, wherein the 1,2-diacyl-2-(t-alkyl) hydrazine is selected from: N'-*t*-butyl-N,N'-dibenzoylhydrazine, N'-*t*-butyl-N-(4-chlorobenzoyl)-N'-benzoylhydrazine, N'-*t*-butyl-N-(4-ethylbenzoyl)-N'-(3,5-dimethylbenzoyl)hydrazine, N'-*t*-butyl-N-(2-methyl-3-methoxybenzoyl)-N'-(3,5-dimethylbenzoyl)hydrazine; N'-*t*-butyl-N-(4-ethylbenzoyl)-N'-(3-methylbenzoyl)hydrazine, N'-*t*-butyl-N-(2-ethyl-3-methoxybenzoyl)-N'-(3,5-dimethylbenzoyl)hydrazine, N'-*t*-butyl-N-(2,3-dimethylbenzoyl)-N'-(3,5-dimethylbenzoyl)hydrazine, N'-*t*-butyl-N-(2,3-dimethylbenzoyl)-N'-(3-methylbenzoyl)hydrazine, N'-*t*-butyl-N-(4-ethylbenzoyl)-N'-(3,5-dichlorobenzoyl)hydrazine, N'-*t*-butyl-N-(2-methyl-3,4-(1,2-ethylenedioxy)benzoyl)-N'-(3,5-dimethylbenzoyl)hydrazine, N'-*t*-butyl-N-(2-methyl-3,4-(methylenedioxy)benzoyl-N'-(3,5-dimethylbenzoyl)hydrazine, N'-*t*-butyl-N-(2-fluorobenzoyl)-N'-benzoylhydrazine, N'-*t*-butyl-N-(1-naphthoyl)-N'-(3,5-dimethylbenzoyl)hydrazine, N'-(1,1-dimethylpentyl)-N-(4-chlorobenzoyl)-N'-benzoylhydrazine and N'-(1,1,3,3-tetramethylbutyl)-N-(2-methyl-3-methoxybenzoyl)-N'-(3,5-dimethylbenzoyl)hydrazine.

5. A process as claimed in claim 1, utilizing a solvent comprising a (C₂-C₄)alkyl (C₂-C₅)alkanoate ester or a mixture of a (C₂-C₄)alkyl (C₂-C₅)alkanoate ester and water, for the preparation of 1,2-diacyl-2-(t-alkyl)hydrazine compounds of the formula: 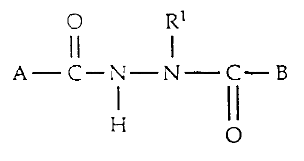 wherein:
R¹ is *t*-butyl, 1,1-dimethylpentyl or 1,1,3,3-tetramethylbutyl;
A and B are each independently (i) phenyl, (ii) naphthyl, or (iii) phenyl or naphthyl substituted with one to three of the same or different substituents selected from the group consisting of: halo; cyano; nitro; thiocyanato; (C₁-C₄)alkyl; (C₁-C₄)alkoxy; halo(C₁-C₂)alkyl; halo(C₁-C₂)alkoxy; (C₁-C₄)alkylthio; (C₁-C₄)alkylsulfinyl; (C₁-C₄)alkylsulfonyl; carboxy; formyl; (C₁-C₄)alkylcarbonyl; (C₁-C₄)alkoxycarbonyl; (C₁-C₄)alkanoyloxy; di(C₁-C₄)alkylamino having independently the stated number of carbon atoms in each alkyl group; carbamoyl; (C₁-C₄)alkylcarbamoyl; di(C₁-C₄)alkylcarbamoyl having independently the stated number of carbon atoms in each alkyl group; cyano(C₁-C₄)alkyl; (C₁-C₄)alkoxy(C₁-C₄)alkyl; (C₂-C₆)alkenyl; (C₄-C₆)alkadienyl; (C₂-C₆)alkynyl; (C₁-C₄)alkyldithionate; (C₁-C₄)alkylcarbonylthio; tri(C₁-C₄)alkylsilyl having independently the stated number of carbon atoms in each alkyl group; phenyl; phenoxy; benzoyl; phenyl(C₁-C₂)alkyl; or phenyl(C₁-C₂)alkyl substituted on the phenyl ring with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, di(C₁-C₄)alkylamino having independently the stated number of carbon atoms in each alkyl group; or when two adjacent positions on a phenyl ring are substituted with alkoxy groups, these groups may be joined to form a 5 or 6 membered dioxolano or dioxano heterocyclic ring;
or the agronomically acceptable salts thereof;
whereby said process comprises:
(a) reacting an aromatic acid chloride of the formula: 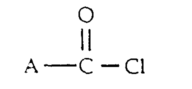 with *t*-butylhydrazine, 1,1-dimethylpentylhydrazine or 1,1,3,3-tetramethylbutylhydrazine or a corresponding acid addition salt of said hydrazine in the presence of potassium hydroxide, sodium hydroxide, sodium carbonate, potassium carbonate, sodium bicarbonate, potassium bicarbonate or a mixture thereof at a temperature of from -5° C to 40° C to form an interrnediate 1-acyl-2-(*t*-alkyl)hydrazine of the formula: 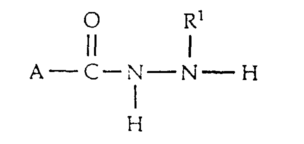 wherein A and R¹ have the same definitions as previously; and
(b) reacting said intermediate hydrazine from step (a) with an aromatic acid chloride of the formula: 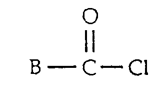 in the presence of potassium hydroxide, sodium hydroxide, sodium carbonate, potassium carbonate, sodium bicarbonate, potassium bicarbonate or a mixture thereof at a temperature of from 25° C to 95° C to form the said 1,2-diacyl-2-(*t*-alkyl)hydrazine, wherein B has the same definitions as previously.

6. A process as claimed in claim 5, utilizing a solvent comprising a mixture of water and an ester selected from the group consisting of isobutyl acetate, ethyl acetate, isopropyl acetate and *n*-butyl acetate, for the preparation of 1,2-diacyl-2-(*t*-butyl)hydrazine compounds of the formula: 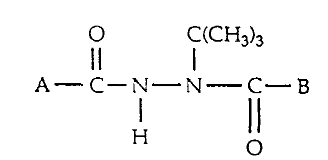 wherein:
A and B are each independently phenyl, or phenyl substituted with one to three of the same or different substituents selected from the group consisting of halo, (C₁-C₂)alkyl, (C₁-C₂)alkoxy, (C₁-C₂)alkoxy(C₁-C₂)alkyl ,or when two adjacent positions on a phenyl ring are substituted with alkoxy groups, these groups may be joined to form a 5 or 6 membered dioxolano or dioxano heterocyclic ring;
whereby said process comprises:
(a) reacting an aromatic acid chloride of the formula: 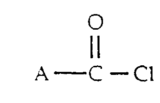 with *t*-butylhydrazine or *t*-butylhydrazine hydrochloride in the presence of potassium hydroxide, sodium hydroxide, sodium carbonate, sodium bicarbonate, potassium carbonate, or a mixture thereof to form an intermediate 1-acyl-2-butyl)hydrazine of the formula: 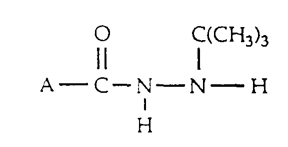 wherein A has the same definitions as previously; and
(b) reacting said intermediate hydrazine from step (a) with an aromatic acid chloride of the formula: 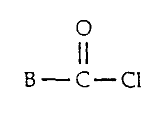 in the presence of sodium carbonate, sodium bicarbonate, potassium carbonate or a mixture thereof to form the said 1,2-diacyl-2-(*t*-butyl)hydrazine, wherein B has the same definitions as previously.

7. A process as claimed in claim 6, utilizing a solvent comprising a mixture of water and n-butyl acetate , for the preparation of 1,2-diacyl-2-(*t*-butyl)hydrazine compounds of the formula: 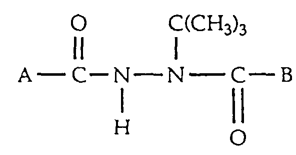 wherein:
A is phenyl, 2-fluorophenyl, 4-chlorophenyl, 2-methyl-3-methoxyphenyl or 4-ethylphenyl;
B is phenyl, 3,5-dimethylphenyl, 3-methylphenyl, 3-chlorophenyl or 3,5-dichlorophenyl;
whereby said process comprises:
(a) reacting an aromatic acid chloride of the formula: 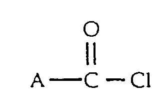 with *t*-butylhydrazine hydrochloride in the presence of a mixture of sodium hydroxide and potassium carbonate to form an intermediate 1-acyl-2-(*t*-butyl)hydrazine of the formula: 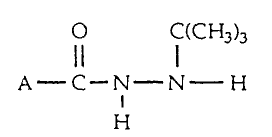 wherein A has the same definitions as previously; and
(b) reacting said intermediate hydrazine from step (a) with an aromatic acid chloride of the formula: 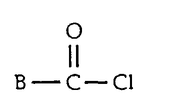 in the presence of sodium carbonate or potassium carbonate to form the said 1,2-diacyl-2-(*t*-butyl)hydrazine, wherein B has the same definitions as previously.

8. A process as claimed in claim 7, wherein the 1,2-diacyl-2-(t-butyl) hydrazine is selected from the group consisting of:
N'-*t*-butyl-N,N'-dibenzoylhydrazine,
N'-*t*-butyl-N-(2-methyl-3-methoxybenzoyl)-N'-(3,5-dimethylbenzoyl)hydrazine,
N'-*t*-butyl-N-(4-ethylbenzoyl)-N'-(3,5-dimethylbenzoyl)hydrazine and
N'-*t*-butyl-N-(4-chlorobenzoyl)-N'-benzoylhydrazine.

## Patentansprüche

1. Verfahren zur Herstellung von 1,2-Diacyl-2-(t-alkyl)-Hydrazin-Pestizidverbindungen der Formel: 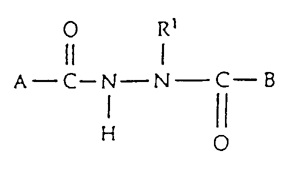 wobei
R¹ eine tertiäre (C₄-C₈)Alkylgruppe ist;
A und B sind jeweils unabhängig voneinander (i) Phenyl, (ii) Naphthyl, oder (iii) Phenyl oder Naphthyl sind, substituiert mit einem bis drei gleichen oder verschiedenen Substituenten, ausgewählt aus Halogen, Cyano, Nitro, Hydroxy, Mercapto, Thiocyanat; (C₁-C₄)Alkyl, (C₁-C₄)Alkoxy, Halogen(C₁-C₂)alkyl, Halogen(C₁-C₂)alkoxy, (C₁-C₄)Alkylthio, (C₁-C₄)Alkylsulfinyl, (C1-C₄)Alkylsulfonyl, Carboxy, Formyl, (C₁-C₄)Alkylcarbonyl, (C₁-C₄)Alkoxycarbonyl, (C₁-C₄)Alkanoyloxy, Amino, (C₁-C₄)Alkylamino, di(C₁-C₄)Alkylamino, das unabhängig voneinander die genannte Anzahl von Kohlenstoffatomen in jeder Alkylgruppe aufweist, Carbamoyl, (C₁-C₄)Alkylcarbamoyl, di(C₁-C₄)Alkylcarbamoyl, das unabhängig voneinander die genannte Zahl an Kohlenstoffatomen in jeder Alkylgruppe aufweist, Cyano(C₁-C₄)alkyl, (C₁-C₄)Alkoxy(C₁-C₄)alkyl, (C₂-C₆)Alkenyl, (C₄-C₆)Alkadienyl, (C₂-C₆)Alkinyl, (C₁-C₄)Alkyldithionat, (C₁-C₄)Alkylcarbonylthio, tri(C₁-C₄)Alkylsilyl, das unabhängig voneinander die genannte Anzahl der Kohlenstoffatome in jeder Alkylgruppe aufweist, Phenyl, Phenoxy, Benzoyl, Phenoxycarbonyl, Phenylthio, Phenyl(C₁-C₄)alkyl, wobei die Phenyl, Phenoxy, Benzoyl, Phenoxycarbonyl, Phenylthio und Phenyl(C₁-C₄)Alkyl-substituenten gegebenenfalls mit einem oder zwei gleichen oder verschiedenen Substituenten substituiert sind, ausgewählt aus Halogen, Cyano, Nitro, Hydroxy, (C₁-C₄)Alkyl, (C₁-C₄)Alkoxy, Halogen(C₁-C₂)alkyl, Halogen(C₁-C₂)alkoxy, (C₁-C₄)Alkylthio, (C₁-C₄)Alkylsulfinyl, (C₁-C₄)Alkylsulfonyl, Carboxy, Formyl, (C₁-C₄)Alkylcarbonyl, (C₁-C₄)Alkoxycarbonyl, (C₁-C₄)Alkanoyloxy, Amino, (C₁-C₄)Alkylamino, di(C₁-C₄)Alkylamino, das unabhängig voneinander die genannte Anzahl von Kohlenstoffatomen in jeder Alkylgruppe aufweist; oder, wenn zwei angrenzende Positionen an einem Phenylring mit Alkoxygruppen substituiert sind, können diese Gruppen verbunden werden, um einen 5- oder 6-gliedrigen Dioxolan (methylendioxy)- oder Dioxolan(1,2-ethylendioxy)-heterozyklischen Ring zu bilden, oder landwirtschaftlich annehmbare Salze davon;
wobei das Verfahren
(a) Umsetzen eines aromatischen Säurechlorids der Formel: 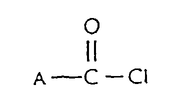 mit einem tertiären (C₄-C₈)Alkylhydrazin der Formel:
H₂NNHR¹
oder einem entsprechenden Säureadditionssalz des tertiären (C₄-C₈)Alkylhydrazins in Gegenwart einer Base, bei einer Temperatur von -20°C bis +40°C, um ein intermediäres 1-Acyl-2-(t-alkyl)hydrazin der Formel: 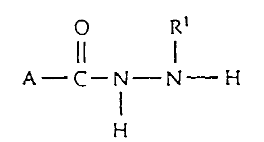 zu bilden,
wobei A und R¹ die gleiche, vorstehend genannten Definitionen aufweisen, und
(b) Umsetzen des intermediären Hydrazin aus Stufe (a) mit einem aromatischen Säurechlorid der Formel: 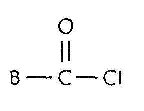 in Gegenwart einer Base bei einer Temperatur von 25°C bis 100°C, um das 1,2-Diacyl-2-(t-alkyl)hydrazin zu bilden, wobei B die gleichen, vorstehend genannten Definitionen aufweist,
umfaßt, und dadurch gekennzeichnet ist, daß das Verfahren unter Verwendung eines Lösungsmittels, umfassend einen Ester oder eine Mischung aus einem Ester und Wasser, ausgeführt wird.

2. Verfahren nach Anspruch 1, wobei das Säurechlorid, das in Stufe (a) und/oder Stufe (b) verwendet wird, ausgewählt ist aus Benzoylchlorid, 3-Methylbenzoylchlorid, 3,5-Dimethylbenzoylchlorid, 3,5-Dichlorbenzoylchlorid, 4-Ethylbenzoylchlorid, 4-Chlorbenzoylchlorid, 2-Methyl-3-methoxybenzoylchlorid, 3,5-Dichlorbenzoylchlorid, 3-Chlorbenzoylchlorid, 4-Cyanobenzoylchlorid, 3-Nitrobenzoylchlorid, 2,3-Dimethylbenzoylchlorid, 2-Methoxybenzoylchlorid, 3-Fluorbenzoylchlorid, 4-(t-Butyl)benzoylchlorid, 2,4-Dichlorbenzoylchlorid, 3-(Trifluormethyl)benzoylchlorid, 2-Acetoxybenzoylchlorid, 4-(4-Chlorphenethyl)benzoylchlorid, 1-Naphthoylchlorid, 4-lsopropylbenzoylchlorid, 2,6-Difluorbenzoylchlorid, 2-(Difluormethoxy)benzoylchlorid, 4-Acetlybenzoylchlorid, 3-(Dimethylamino)benzoylchlorid, 2-Nitro-4-((4-trifluormethyl)-2-chlorphenoxy)benzoylchlorid, Phenylthiobenzoylchlorid, 4-(2-Fluorbenzyl)benzoylchlorid, 4-(4-Chlorbenzoyl)chlorid, 4-(Phenoxycarbonyl)benzoylchlorid, 2-Methyl-3,4-(1,2-ethylendioxy)benzoylchlorid und 2-Methyl-3,4-(methylendioxy)benzoylchlorid.

3. Verfahren nach Anspruch 1 oder Anspruch 2, wobei das tertiäre (C₄-C₈)Alkylhydrazin, das in Stufe (a) verwendet wird, ausgewählt ist aus t-Butylhydrazin, 1,1-Dimethylpentylhydrazin und 1,1,3,3-Tetramethylbutylhydrazin und ihren entsprechenden Säureadditionssalzen.

4. Verfahren nach Anspruch 1, wobei das 1,2-Diacyl-2-(t-alkyl)hydrazin ausgewählt ist aus N'-t-Butyl-N,N'-dibenzoylhydrazin, N'-t-Butyl-N-(4-chlorbenzoyl)-N'-benzoylhydrazin, N'-t-Butyl-N-(4-ethylbenzoyl)-N'-(3,5-dimethylbenzoyl)hydrazin, N'-t-Butyl-N-(2-methyl-3-methoxybenzoyl)-N'-(3,5-dimethylbenzoyl)hydrazin, N'-t-Butyl-N-(4-ethylbenzoyl)-N'-(3-methylbenzoyl)hydrazin, N'-t-Butyl-N-(2-ethyl-3-methoxybenzoyl)-N'-(3,5-dimethylbenzoyl)hydrazin, N'-t-Butyl-N-(2,3-dimethylbenzoyl)-N'-(3,5-dimethylbenzoyl)hydrazin, N'-t-Butyl-N-(2,3-dimethylbenzoyl)-N'-(3-methylbenzoyl)hydrazin, N'-t-Butyl-N-(4-ethylbenzoyl)-N'-(3,5-dichlorbenzoyl)hydrazin, N'-t-Butyl-N-(2-methyl-3,4-(1,2-ethylendioxy)benzoyl)-N'-(3,5-dimethylbenzoyl)hydrazin, N'-t-Butyl-N-(2-methyl-3,4-(methylendioxy)benzoyl-N'-(3,5-dimethylbenzoyl)hydrazin, N'-t-Butyl-N-(2-fluorbenzoyl)-N'-benzoylhydrazin, N'-t-Butyl-N-(1-naphthoyl)-N'-(3,5-dimethylbenzoyl)hydrazin, N'-(1,1-Dimethylpentyl)-N-(4-chlorbenzoyl)-N'-benzoylhydrazin und N'-(1,1,3,3-Tetramethylbutyl)-N-(2-methyl-3-methoxybenzoyl)-N'-(3,5-dimethylbenzoyl)hydrazin.

5. Verfahren nach Anspruch 1, welches ein Lösungsmittel, umfassend einen (C₂-C₄)Alkyl(C₂-C₅)alkanoatester oder eine Mischung aus einem (C₂-C₄) Alkyl(C₂-C₅)alkanoatester und Wasser, zur Herstellung von 1,2-Diacyl-2-(t-alkyl)hydrazin-Verbindungen der Formel: 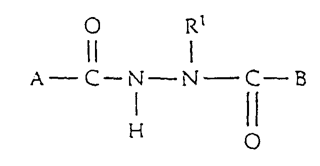 verwendet, wobei
R¹ t-Butyl, 1,1-Dimethylpentyl oder 1,1,3,3-Tetramethylbutyl ist;
A und B jeweils unabhängig voneinander (i) Phenyl, (ii) Naphthyl, oder (iii) Phenyl oder Naphthyl sind, substituiert mit einem bis drei gleichen oder unterschiedlichen Substituenten, ausgewählt aus Halogen, Cyano, Nitro, Thiocyanato, (C₁-C₄)Alkyl, (C₁-C₄)Alkoxy, Halogen(C₁-C₂)alkyl, Halogen (C₁-C₂)alkoxy, (C₁-C₄)Alkylthio, (C₁-C₄)Alkylsulfinyl, (C₁-C₄)Alkylsulfonyl, Carboxy, Formyl, (C₁-C₄)Alkylcarbonyl, (C₁-C₄)Alkoxycarbonyl, (C₁-C₄) Alkanoyloxy, di(C₁-C₄)Alkylamino, das unabhängig voneinander die genannte Anzahl von Kohlenstoffatomen in jeder Alkylgruppe aufweist, Carbamoyl, (C₁-C₄)Alkylcarbamoyl, di(C₁-C₄)Alkylcarbamoyl, das unabhängig voneinander die genannte Zahl an Kohlenstoffatomen in jeder Alkylgruppe aufweist, Cyano(C₁-C₄)alkyl, (C₁-C₄)Alkoxy(C₁-C₄)alkyl, (C₂-C₆)Alkenyl, (C₄-C₆)Alkadienyl, (C₂-C₆)Alkinyl, (C₁-C₄)Alkyldithionat, (C₁-C₄)Alkylcarbonylthio, tri(C₁-C₄)Alkylsilyl, das unabhängig voneinander die genannte Anzahl der Kohlenstoffatome in jeder Alkylgruppe aufweist, Phenyl, Phenoxy, Benzoyl, Phenyl(C₁-C₂)alkyl, oder Phenyl(C₁-C₂)alkyl, welches am Phenylring mit einem bis zwei gleichen oder verschiedenen Substituenten substituiert ist, welche ausgewählt sind aus Halogen, Cyano, Nitro, Hydroxy, (C₁-C₄)Alkyl, (C₁-C₄)Alkoxy, Halogen(C₁-C₂)alkyl, Halogen(C₁-C₂)alkoxy, (C₁-C₄)Alkylthio, (C₁-C₄)Alkylsulfinyl, (C₁-C₄)Alkylsulfonyl, Carboxy, Formyl, (C₁-C₄)Alkylcarbonyl, (C₁-C₄)Alkoxycarbonyl, (C₁-C₄)Alkanoyloxy, di(C₁-C₄)Alkylamino, das unabhängig voneinander die angegebene Anzahl von Kohlenstoffatomen in jeder Alkylgruppe aufweist, oder, wenn zwei angrenzende Positionen an einem Phenylring mit Alkoxygruppen substituiert sind, können diese Gruppen verbunden werden, um einen 5- oder 6-gliedrigen Dioxolan- oder Dioxan-heterozyklischen Ring zu bilden;
oder landwirtschaftlich annehmbare Salze davon,
wobei das Verfahren
(a) Umsetzen eines aromatischen Säurechlorids der Formel: 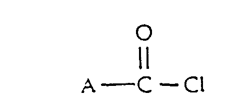 mit t-Butylhydrazin, 1,1-Dimethylpentylhydrazine oder 1,1,3,3-Tetramethylbutylhydrazin oder einem entsprechenden Säureadditionssalz des Hydrazins in Gegenwart von Kaliumhydroxid, Natriumhydroxid, Natriumcarbonat, Kaliumcarbonat, Natriumbicarbonat, Kaliumbicarbonat oder einer Mischung davon bei einer Temperatur von -5°C bis 40°C, um ein intermediäres 1-Acyl-2-(t-alkyl)hydrazin der Formel: 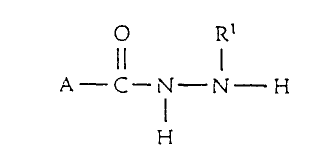 zu bilden, wobei A und R¹ die gleichen, vorstehend genannten Definitionen aufweisen, und
(b) Umsetzen des intermediären Hydrazins aus Stufe (a) mit einem aromatischen Säurechlorid der Formel: 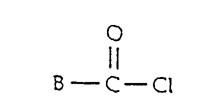 in Gegenwart von Kaliumhydroxid, Natriumhydroxid, Natriumcarbonat, Kaliumcarbonat, Natriumbicarbonat, Kaliumbicarbonat oder einer Mischung davon bei einer Temperatur von 25°C bis 95°C, um das 1,2-Diacyl-2-(t-alkyl)hydrazin zu bilden, wobei B die gleichen, vorstehend genannten Definitionen aufweist, umfaßt.

6. Verfahren nach Anspruch 5, welches ein Lösungsmittel, umfassend eine Mischung aus Wasser und einem Ester, ausgewählt aus Isobutylacetat, Ethylacetat, Isopropylacetat und n-Butylacetat, zur Herstellung von 1,2-Diacyl-2-(t-butyl)hydrazin-Verbindungen der Formel: 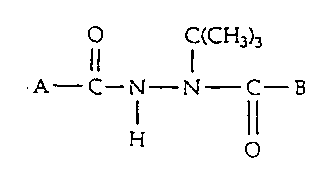 verwendet, wobei:
A und B jeweils unabhängig voneinander Phenyl oder Phenyl, substituiert mit einem bis drei gleichen oder verschiedenen Substituenten, ausgewählt aus Halogen, (C₁-C₂)Alkyl, (C₁-C₂)Alkoxy, (C₁-C₂)Alkoxy(C₁-C₂)alkyl, oder, wenn zwei angrenzende Positionen an einem Phenylring mit Alkoxygruppen substituiert sind, können diese Gruppen verbunden werden, um einen 5- oder 6-gliedrigen Dioxolan- oder Dioxan-heterozyklischen Ring zu bilden, sind,
wobei das Verfahren
(a) Umsetzen eines aromatischen Säurechlorid der Formel: 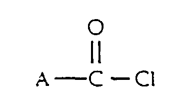 mit t-Butylhydrazin oder t-Butylhydrazinhydrochlorid in Gegenwart von Kaliumhydroxid, Natriumhydroxid, Natriumcarbonat, Natriumbicarbonat, Kaliumcarbonat oder einer Mischung davon, um ein intermediäres 1-Acyl-2-(t-butyl)hydrazin der Formel: 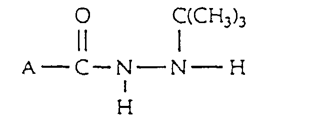 zu bilden, wobei A die gleichen, vorstehend genannten Definitionen aufweist, und
(b) Umsetzen des intermediären Hydrazin aus Stufe (a) mit einem aromatischen Säurechlorid der Formel: 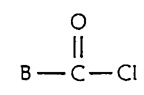 in Gegenwart von Natriumcarbonat, Natriumbicarbonat, Kaliumcarbonat oder einer Mischung davon, um das 1,2-Diacyl-2-(t-butyl)hydrazin zu bilden, wobei B die gleichen, vorstehend genannten Definitionen aufweist, umfaßt.

7. Verfahren nach Anspruch 6, welches ein Lösungsmittel, umfassend eine Mischung aus Wasser und n-Butylacetat, zur Herstellung von 1,2-Diacyl-2-(t-butyl)hydrazinverbindungen der Formel: 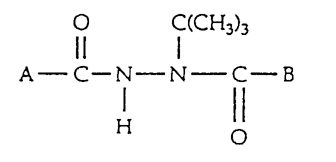 verwendet, wobei
A Phenyl, 2-Fluorphenyl, 4-Chlorphenyl, 2-Methyl-3-methoxyphenyl oder 4-Ethylphenyl ist;
B Phenyl, 3,5-Dimethylphenyl, 3-Methylphenyl, 3-Chlorphenyl oder 3,5-Dichlorphenyl ist;
wobei das Verfahren
(a) Umsetzen eines aromatischen Säurechlorids der Formel: 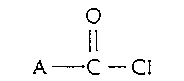 mit t-Butylhydrazinhydrochlorid in Gegenwart einer Mischung aus Natriumhydroxid und Kaliumcarbonat, um ein intermediäres 2-Acyl-2-(t-butyl)hydrazin der Formel 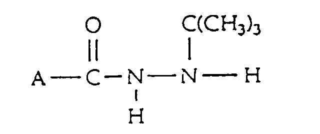 zu bilden, wobei A die gleichen, vorstehend genannten Definitionen aufweist, und
(b) Umsetzen des intermediären Hydrazins aus Stufe (a) mit einem aromatischen Säurechlorid der Formel: 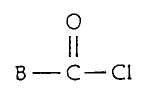 in Gegenwart von Natriumcarbonat oder Kaliumcarbonat, um das 1,2-Diacyl-2-(t-butyl)hydrazin zu bilden, wobei B die gleichen, vorstehend genannten Definitionen aufweist, umfaßt.

8. Verfahren nach Anspruch 7, wobei das 1,2-Diacyl-2-(t-butyl)hydrazin ausgewählt ist aus
N'-t-Butyl-N,N'-dibenzoylhydrazin,
N'-t-Butyl-N-(2-methyl-3-methoxybenzoyl)-N'-(3,5-dimethylbenzoyl)hydrazin,
N'-t-Butyl-N-(4-ethylbenzoyl)-N'-(3,5-dimethylbenzoyl)hydrazin und
N'-t-Butyl-N-(4-chlorbenzoyl)-N'-benzoylhydrazin.

## Revendications

1. Procédé pour la préparation de composés pesticides 1,2-diacyl-2-(t-alkyl)hydrazines de formule: 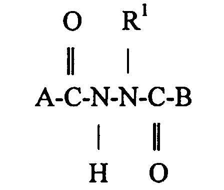 dans laquelle
R¹ est un groupe alkyle tertiaire en C₄-C₈,
A et B sont chacun indépendamment (i) un groupe phényle, (ii) un groupe naphtyle ou (iii) un groupe phényle ou naphtyle substitué par 1-3 substituants, identiques ou différents, choisis dans l'ensemble constitué par les groupes halogéno, cyano, nitro, hydroxy, mercapto, thiocyanato, alkyle en C₁-C₄, alcoxy en C₁-C₄, halogénoalkyle en C₁-C₂, halogénoalcoxy en C₁-C₂, alkylthio en C₁-C₄, alkylsulfinyle en C₁-C₄, alkylsulfonyle en C₁-C₄, carboxy, formyle, (alkyle en C₁-C₄)carbonyle, (alcoxy en C₁-C₄)carbonyle, alcanoyloxy en C₁-C₄, amino, (alkyle en C₁-C₄)amino, di(alkyle C₁-C₄)amino ayant indépendamment le nombre indiqué d'atomes de carbone dans chaque groupe alkyle, carbamoyle, (alkyle en C₁-C₄)carbamoyle, di(alkyle en C₁-C₄)carbamoyle ayant indépendamment le nombre indiqué d'atomes de carbone dans chaque groupe alkyle, cyanoalkyle en C₁-C₄, (alcoxy en C₁-C₄)(alkyle en C₁-C₄), alcényle en C₂-C₆, alcadiényle en C₄-C₆, alcynyle en C₂-C₆, alkyldithionate en C₁-C₄, (alkyle en C₁-C₄)carbonylthio, tri(alkyle en C₁-C₄)silyle ayant indépendamment le nombre indiqué d'atomes de carbone dans chaque groupe alkyle, phényle, phénoxy, benzoyle, phénoxycarbonyle, phénylthio, phényl(alkyle en C₁-C₄), et dans lequel les substituants phényle, phénoxy, benzoyle, phénoxycarbonyle, phénylthio et phényl(alkyle en C₁-C₄) sont éventuellement substitués par 1 ou 2 substituants, identiques ou différents, choisis dans l'ensemble comprenant les groupes halogéno, cyano, nitro, hydroxy, alkyle en C₁-C₄, alcoxy en C₁-C₄, halogénoalkyle en C₁-C₂, halogénoalcoxy en C₁-C₂, alkylthio en C₁-C₄, alkylsulfinyle en C₁-C₄, alkylsulfonyle en C₁-C₄, carboxy, formyle, (alkyle en C₁-C₄)carbonyle, (alcoxy en C₁-C₄)carbonyle, alcanoyloxy en C₁-C₄, amino, (alkyle en C₁-C₄)amino, di(alkyle en C₁-C₄)amino ayant indépendamment le nombre indiqué d'atomes de carbone dans chaque groupe alkyle, ou bien, quand deux positions adjacentes sur un cycle phényle sont substituées par des groupes alcoxy, ces groupes peuvent être réunis pour former un noyau hétérocyclique dioxolanno (méthylènedioxy) ou dioxanno (1,2-éthylènedioxy)-5 ou 6 chaînons;
ou de leurs sels acceptables en agronomie;
ledit procédé consistant:
(a) à faire réagir un chlorure d'acide aromatique de formule: 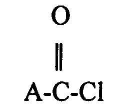 avec une (alkyle tertiaire en C₄-C₈)hydrazine de formule:
H₂NNHR¹
ou un sel d'addition d'acide correspondant de ladite (alkyle tertiaire en C₄-C₈)hydrazine en présence d'une base,-une température comprise entre -20°C et 40°C, pour former un intermédiaire 1-acyl-2-(t-alkyl)hydrazine de formule: 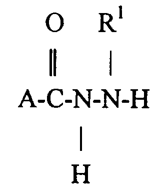 dans laquelle A et R¹ ont les mêmes définitions que précédemment; et
(b) à faire réagir ladite hydrazine intermédiaire de l'étape (a) avec un chlorure d'acide aromatique de formule: 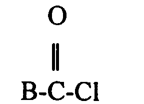 en présence d'une base,-une température comprise entre 25°C et 100°C, pour former ladite 1,2-diacyl-2-(t-alkyl)hydrazine, dans laquelle B a les mêmes définitions que précédemment;
et caractérisé en ce que le procédé se déroule en utilisant un solvant comprenant un ester ou un mélange d'un ester et d'eau.

2. Procédé selon la revendication 1, dans lequel le chlorure d'acide, utilisé dans l'étape (a) et/ou l'étape (b), est choisi parmi le chlorure de benzoyle, le chlorure de 3-méthylbenzoyle, le chlorure de 3,5-diméthylbenzoyle, le chlorure de 3,5-dichlorobenzoyle, le chlorure de 4-éthylbenzoyle, le chlorure de 4-chlorobenzoyle, le chlorure de 2-méthyl-3-méthoxy-benzoyle, le chlorure de 3,5-dichlorobenzoyle, le chlorure de 3-chlorobenzoyle, le chlorure de 4-cyanobenzoyle, le chlorure de 3-nitrobenzoyle, le chlorure de 2,3-diméthylbenzoyle, le chlorure de 2-méthoxybenzoyle, le chlorure de 3-fluorobenzoyle, le chlorure de 4-(t-butyl)benzoyle, le chlorure de 2,4-dichlorobenzoyle, le chlorure de 3-(trifluorométhyl) benzoyle, le chlorure de 2-acétoxybenzoyle, le chlorure de 4-(4-chlorophénéthyl)benzoyle, le chlorure de 1-naphtoyle, le chlorure de 4-isopropylbenzoyle, le chlorure de 2,6-difluorobenzoyle, le chlorure de 2-(difluorométhoxy)benzoyle, le chlorure de 4-acétylbenzoyle, le chlorure de 3-(diméthylamino) benzoyle, le chlorure de 2-nitro-4-((4-trifluorométhyl)-2-chlorophénoxy)benzoyle, le chlorure de phénylthiobenzoyle, le chlorure de 4-(2-fluorobenzyl) benzoyle, le chlorure de 4-(4-chlorobenzoyl)benzoyle, le chlorure de 4-(phénoxycarbonyl)benzoyle, le chlorure de 2-méthyl-3,4-(1,2-éthylènedioxy)benzoyle, et le chlorure de 2-méthyl-3,4-(méthylènedioxy)benzoyle.

3. Procédé selon la revendication 1 ou 2, dans lequel la (alkyle tertiaire en C₄-C₈)hydrazine utilisée dans l'étape (a) est choisie parmi la t-butylhydrazine, la 1,1-diméthylpentylhydrazine et la 1,1,3,3-tétraméthylbutylhydrazine, ainsi que leurs sels d'addition d'acide correspondants.

4. Procédé selon la revendication 1, dans lequel la 1,2-diacyl-2-(t-alkyl)hydrazine est choisie parmi la N'-t-butyl-N,N'-dibenzoylhydrazine, la N'-t-butyl-N-(4-chlorobenzoyl)-N'-benzoylhydrazine, la N'-t-butyl-N-(4-éthylbenzoyl)-N'-(3,5-diméthylbenzoyl)hydrazine, la N'-t-butyl-N-(2-méthyl-3-méthoxybenzoyl)-N'-(3,5-diméthylbenzoyl)hydrazine, la N'-t-butyl-N-(4-éthylbenzoyl)-N'-(3-méthylbenzoyl)hydrazine, la N'-t-butyl-N-(2-éthyl-3-méthoxybenzoyl)-N'-(3,5-diméthylbenzoyl)hydrazine, la N'-t-butyl-N-(2,3-diméthylbenzoyl)-N'-(3,5-diméthylbenzoyl)hydrazine, la N'-t-butyl-N-(2,3-diméthylbenzoyl)-N'-(3-méthylbenzoyl)hydrazine, la N'-t-butyl-N-(4-éthylbenzoyl)-N'-(3,5-dichlorobenzoyl)hydrazine, la N'-t-butyl-N-(2-méthyl-3,4-(1,2-éthylènedioxy)benzoyl)-N'-(3,5-diméthylbenzoyl)hydrazine, la N'-t-butyl-N-(2-méthyl-3,4-(méthylènedioxy)benzoyl)-N'-(3,5-diméthylbenzoyl)hydrazine, la N'-t-butyl-N-(2-fluoro-benzoyl)-N'-benzoylhydrazine, la N'-t-butyl-N-(1-naphtoyl)-N'-(3,5-diméthylbenzoyl)hydrazine, la N'-(1,1-diméthylpentyl)-N-(4-chlorobenzoyl)-N'-benzoylhydrazine et la N'-(1,1,3,3-tétraméthylbutyl)-N-(2-méthyl-3-méthoxybenzoyl)-N'-(3,5-diméthylbenzoyl)hydrazine.

5. Procédé selon la revendication 1, utilisant un solvant comprenant un ester alcanoate en C₂-C₅ d'alkyle en C₂-C₄ ou un mélange d'un ester alcanoate en C₂-C₅ d'alkyle en C₂-C₄ et d'eau, pour la préparation de composés 1,2-diacyl-2-(t-alkyl)hydra-zines de formule: 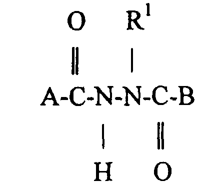 dans laquelle
R¹ est un groupe t-butyle, 1,1-diméthylpentyle ou 1,1,3,3-tétraméthylbutyle;
A et B sont chacun indépendamment (i) un groupe phényle, (ii) un groupe naphtyle ou (iii) un groupe phényle ou naphtyle substitué par 1-3 substituants, identiques ou différents, choisis dans l'ensemble constitué par les groupes halogéno, cyano, nitro, thiocyanato, alkyle en C₁-C₄, alcoxy en C₁-C₄, halogénoalkyle en C₁-C₂, halogénoalcoxy en C₁-C₂, alkylthio en C₁-C₄, alkylsulfinyle en C₁-C₄, alkylsulfonyle en C₁-C₄, carboxy, formyle, (alkyle en C₁-C₄)carbonyle, (alcoxy en C₁-C₄)carbonyle, alcanoyloxy en C₁-C_{4,} di(alkyle C₁-C₄)amino ayant indépendamment le nombre indiqué d'atomes de carbone dans chaque groupe alkyle, carbamoyle, (alkyle en C₁-C₄)carbamoyle, di(alkyle en C₁-C₄)carbamoyle ayant indépendamment le nombre indiqué d'atomes de carbone dans chaque groupe alkyle, cyanoalkyle en C₁-C₄, (alcoxy en C₁-C₄)(alkyle en C₁-C₄), alcényle en C₂-C₆, alcadiényle en C₄-C₆, alcynyle en C₂-C₆, alkyldithionate en C₁-C₄, (alkyle en C₁-C₄)carbonylthio, tri(alkyle en C₁-C₄)silyle ayant indépendamment le nombre indiqué d'atomes de carbone dans chaque groupe alkyle, phényle, phénoxy, benzoyle, phényl(alkyle en C₁-C₂) ou phényl(alkyle en C₁-C₂) substitué sur le cycle phényle par un ou deux substituants, identiques ou différents, choisis dans l'ensemble constitué par les groupes halogéno, cyano, nitro, hydroxy, alkyle en C₁-C₄, alcoxy en C₁-C₄, halogénoalkyle en C₁-C₂, halogénoalcoxy en C₁-C₂, alkylthio en C₁-C₄, alkylsulfinyle en C₁-C₄, alkylsulfonyle en C₁-C₄, carboxy, formyle, (alkyle en C₁-C₄)carbonyle, (alcoxy en C₁-C₄)carbonyle, alcanoyloxy en C₁-C₄, di(alkyle en C₁-C₄)amino ayant indépendamment le nombre indiqué d'atomes de carbone dans chaque groupe alkyle, ou bien, quand deux positions adjacentes sur un cycle phényle sont substituées par des groupes alcoxy, ces groupes peuvent être réunis pour former un noyau hétérocyclique dioxolanno ou dioxanno-5 ou 6 chaînons;
ou de leurs sels acceptables en agronomie;
ledit procédé consistant:
(a) à faire réagir un chlorure d'acide aromatique de formule: 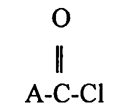 avec de la t-butylhydrazine, de la 1,1-diméthylpentylhydrazine ou de la 1,1,3,3-tétraméthylbutylhydrazine ou un sel d'addition d'acide correspondant de ladite hydrazine en présence d'hydroxyde de potassium, d'hydroxyde de sodium, de carbonate de sodium, de carbonate de potassium, de bicarbonate de sodium, de bicarbonate de potassium ou d'un de leurs mélanges,-une température comprise entre -5°C et 40°C, pour former un intermédiaire 1-acyl-2-(t-alkyl)hydrazine de formule: 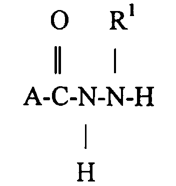 dans laquelle A et R¹ ont les mêmes définitions que précédemment; et
(b) à faire réagir ladite hydrazine intermédiaire de l'étape (a) avec un chlorure d'acide aromatique de formule: 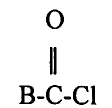 en présence d'hydroxyde de potassium, d'hydroxyde de sodium, de carbonate de sodium, de carbonate de potassium, de bicarbonate de sodium, de bicarbonate de potassium ou d'un de leurs mélanges,-une température comprise entre 25°C et 95°C, pour former ladite 1,2-diacyl-2-(t-alkyl)hydrazine, dans laquelle B a les mêmes définitions que précédemment.

6. Procédé selon la revendication 5, utilisant un solvant comprenant un mélange d'eau et d'un ester choisi dans l'ensemble constitué par l'acétate d'isobutyle, l'acétate d'éthyle, l'acétate d'isopropyle et l'acétate de n-butyle, pour la préparation de composés 1,2-diacyl-2-(t-butyl)hydrazines de formule: 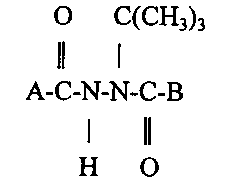 dans laquelle
A et B sont chacun indépendamment un groupe phényle ou groupe phényle substitué par 1-3 substituants, identiques ou différents, choisis dans l'ensemble constitué par les groupes halogéno, alkyle en C₁-C₂, alcoxy en C₁-C₂, et (alcoxy en C₁-C₂)(alkyle en C₁-C₂), ou bien, quand deux positions adjacentes sur un cycle phényle sont substituées par des groupes alcoxy, ces groupes peuvent être réunis pour former un noyau hétérocyclique dioxolanno ou dioxanno-5 ou 6 chaînons;
ledit procédé consistant:
(a) à faire réagir un chlorure d'acide aromatique de formule: 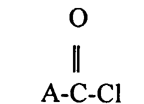 avec de la t-butylhydrazine ou du chlorhydrate de t-butylhydrazine, en présence d'hydroxyde de potassium, d'hydroxyde de sodium, de carbonate de sodium, de bicarbonate de sodium, de carbonate de potassium, ou d'un de leurs mélanges, pour former un intermédiaire 1-acyl-2-(t-butyl)hydrazine de formule: 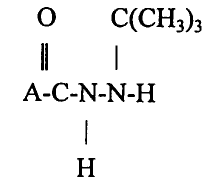 dans laquelle A a les mêmes définitions que précédemment; et
(b) à faire réagir ladite hydrazine intermédiaire de l'étape (a) avec un chlorure d'acide aromatique de formule: 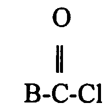 en présence de carbonate de sodium, de bicarbonate de sodium, de carbonate de potassium ou d'un de leurs mélanges, pour former ladite 1,2-diacyl-2-(t-butyl)hydrazine, dans laquelle B a les mêmes définitions que précédemment.

7. Procédé selon la revendication 6, utilisant un solvant comprenant un mélange d'eau et d'acétate de n-butyle en tant que solvant, pour la préparation de composés 1,2-diacyl-2-(t-butyl)hydrazines de formule: 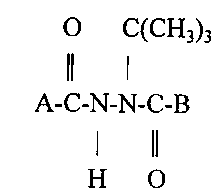 dans laquelle
A est un groupe phényle, 2-fluorophényle, 4-chlorophényle, 2-méthyl-3-méthoxyphényle ou 4-éthylphényle;
B est un groupe phényle, 3,5-diméthylphényle, 3-méthylphényle, 3-chlorophényle ou 3,5-dichlorophényle;
ledit procédé consistant:
(a) à faire réagir un chlorure d'acide aromatique de formule: 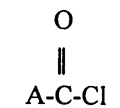 avec du chlorhydrate de t-butylhydrazine en présence d'un mélange d'hydroxyde de sodium et de carbonate de potassium pour former un intermédiaire 1-acyl-2-(t-butyl)hydrazine de formule: 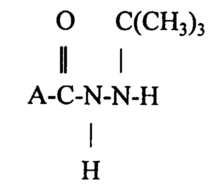 dans laquelle A a les mêmes définitions que précédemment; et
(b) à faire réagir ladite hydrazine intermédiaire de l'étape (a) avec un chlorure d'acide aromatique de formule: 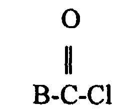 en présence de carbonate de sodium ou de carbonate de potassium pour former ladite 1,2-diacyl-2-(t-butyl)hydrazine, dans laquelle B a les mêmes définitions que précédemment.

8. Procédé selon la revendication 7, dans laquelle la 1,2-diacyl-2-(t-butyl)hydrazine est choisie dans l'ensemble constitué par:
la N'-t-butyl-N,N'-dibenzoylhydrazine,
la N'-t-butyl-N-(2-méthyl-3-méthoxybenzoyl)-N'-(3,5-diméthylbenzoyl)hydrazine,
la N'-t-butyl-N-(4-éthylbenzoyl)-N'-(3,5-diméthylbenzoyl)hydrazine et
la N'-t-butyl-N-(4-chlorobenzoyl)-N'-benzoylhydrazine.
